# EUROPÄISCHE PATENTANMELDUNG

(11) **EP 1 269 929 A1**
(43) Veröffentlichungstag der Anmeldung: **02.01.2003**
(21) Anmeldenummer: 01114917.6
(22) Anmeldetag: 20.06.2001
(51) Int. Cl.: A61B 17/70

(54) **Vergurtungssystem zur Spondylodese der Lendenwirbelsäule**

(71) Anmelder: Copf jun., Franz, D-70178 Stuttgart (DE)
(72) Erfinder: Copf jun., Franz, D-70178 Stuttgart (DE)
(74) Vertreter: Ostertag, Ulrich, Dr.

(57) **Zusammenfassung**

Ein Vergurtungssystem (10) zur Spondylodese der Lendenwirbelsäule umfaßt mindestens zwei jeweils in einem Pedikel verankerbare Verankerungselemente (12) und mindestens eine zwischen zwei benachbarten Wirbelkörpern (74, 76) verlaufende Verbindungsstange (14). Für jedes Verankerungselement (12) ist eine über ein Befestigungselement (58) an diesem befestigbare Verbindungseinrichtung (16) für die Verbindungsstange (16) vorgesehen. Zur Vereinfachung der Operation wird vorgeschlagen, daß die Verbindungseinrichtung (16) ein mit dem Verankerungselement (12) in axialer Richtung verbindbares Befestigungsteil (32) aufweist, das einen seitlichen Lagerabschnitt (36) besitzt, in dem ein Endabschnitt der Verbindungsstange (14) fest aufgenommen werden kann.

## Beschreibung

Die vorliegende Erfindung betrifft ein Vergurtungssystem zur Spondylodese der Lendenwirbelsäule nach dem Oberbegriff des Anspruches 1.

Ein derartiges vom Markt her bekanntes Vergurtungssystem wird zur Spondylodese der Lendenwirbelsäule, d.h. zur starren Verbindung zweier benachbarter Wirbelkörper z.B. beschädigter Bandscheibe, eingesetzt. Es umfaßt zwei Pedikelschrauben, wobei die eine Pedikelschraube im Pedikel des einen Wirbelkörpers und die andere Pedikelschraube im Pedikel des benachbarten Wirbelkörpers verankert werden kann, sowie eine an den beiden Pedikelschrauben befestigbare Verbindungsstange.

Nachteilig bei dem bekannten Vergurtungssystem ist jedoch, daß für seinen Einsatz die paravertebrale Muskulatur über eine Strecke von 15 bis 20 cm von der Wirbelsäule präpariert werden muß, und zwar über die Gelenkfortsätze lateral hinaus. Dabei wird die neurale und vaskuläre Versorgung in diesem Bereich weitgehend unterbrochen. Die relativ große Wunde bleibt über einen Zeitraum von ca. 1 1/2 bis 2 1/2 Stunden offen, was zu einem entsprechenden Infektionsrisiko führt. Zuzüglich muß bei einer solchen großen Wunde mit einem Blutverlust von ca. 300 bis 500 ml gerechnet werden, was für den Kreislauf des Patienten belastend ist. Daher sollte die Operationswunde möglichst klein gehalten werden, wodurch aber auch das Gesichtsfeld des Operateurs klein wird.

Die vorliegende Erfindung hat daher die Aufgabe, ein Vergurtungssystem der eingangs genannten Art so weiterzubilden, daß das Setzen des Vergurtungssystems auch bei kleinem Gesichtsfeld möglich ist.

Diese Aufgabe wird durch ein Vergurtungssystem mit den im Anspruch 1 angegebenen Merkmalen gelöst.

Bei dem erfindungsgemäßen Vergurtungssystem ist es nicht mehr notwendig, zum Herstellen der Vergurtung eine große Wunde im Bereich der zu verbindenden Wirbelkörper zu schaffen. Da das erfindungsgemäß vorgesehene Befestigungsteil mit dem Verankerungselement in axialer Richtung verbindbar ist, können die Arbeiten zum Herstellen der Vergurtung "von oben" in einem relativ engen Kanal durchgeführt werden. Durch die axiale Verbindbarkeit bedingt ist der Lagerabschnitt für die Verbindungsstange seitlich angeordnet. Auf diese Weise kann die Größe der für den Eingriff notwendigen Wunde erheblich verkleinert werden.

Vorteilhafte Weiterbildungen der Erfindung sind in Unteransprüchen angegeben.

Die Weiterbildung nach Anspruch 2 hat den Vorteil, daß die Verbindungsstange ebenfalls relativ leicht "von oben" an der Verbindungseinrichtung befestigt werden kann.

Bei der in Anspruch 3 angegebenen Klemmschraube handelt es sich um ein einfaches Klemmelement, welches den Verbindungsstange sicher und dauerhaft in der U-förmigen Querschnitt aufweisenden Ausnehmung festlegen kann.

Der Aufbau der erfindungsgemäßen Vergurtung wird durch die in Anspruch 4 vorgesehene Führungsstange erleichtert.

Das Anbringen des Befestigungsteils an der Pedikelschraube wird durch die in Anspruch 5 angegebene Weiterbildung erleichtert, da mit ihr ein "Auffädeln" des Befestigungsteils auf den Kopf der Pedikelschraube möglich ist.

Eine weitere Erleichterung beim Aufbau des erfindungsgemäßen Vergurtungssystems bietet die in Anspruch 6 angesprochene Führungshülse.

Das Befestigungselement, mit dem das Befestigungsteil an der Pedikelschraube befestigt wird, und/oder das Klemmelement, mit dem die Verbindungsstange am Befestigungsteil festgelegt wird, können bei der Weiterbildung nach Anspruch 7 auf einfache Weise ebenfalls "von oben", also in axialer Richtung des Verankerungselements, in ihre entsprechende Einbauposition gebracht werden.

Wichtig für den einfachen Zusammenbau des erfindungsgemäßen Vergurtungssystems sind auch die in Anspruch 8 angegebenen Einführschrägen, da sie eine "Zielhilfe" für die einzuführenden Teile darstellen.

Eine noch präzisere Ausrichtung des Vergurtungssystems und eine einfache Anpassung an individuelle Gegebenheiten ist durch die Weiterbildung gemäß Anspruch 9 möglich.

Nachfolgend wird ein Ausführungsbeispiel der Erfindung unter Bezugnahme auf die Zeichnung im Detail erläutert. In dieser zeigen:
- Figur 1:: einen Längsschnitt durch eine Verbindungseinrichtung eines Vergurtungssystems zu einem ersten Zeitpunkt des Zusammenbaus;
- Figur 2:: einen Längsschnitt durch die Verbindungseinrichtung von Figur 1 zu einem zweiten Zeitpunkt des Zusammenbaus; und
- Figur 3:: eine grobschematische Darstellung des Operationsgebiets nach erfolgter Vergurtung von außen gesehen.

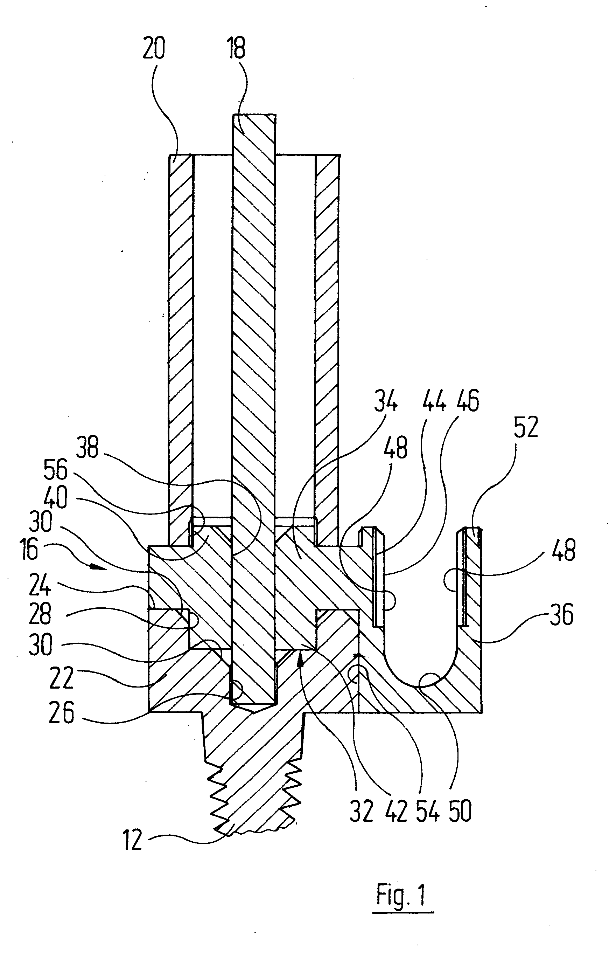

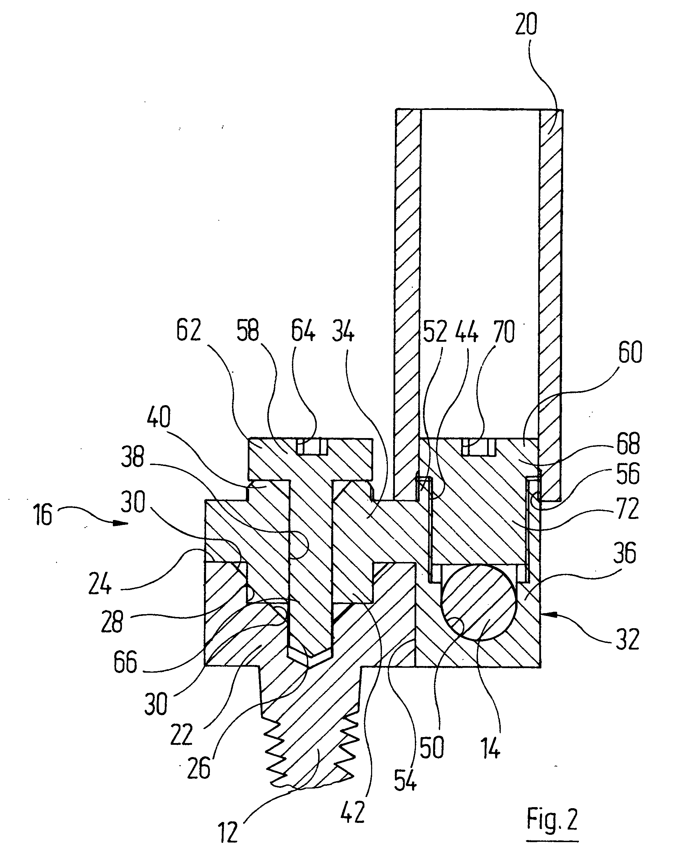

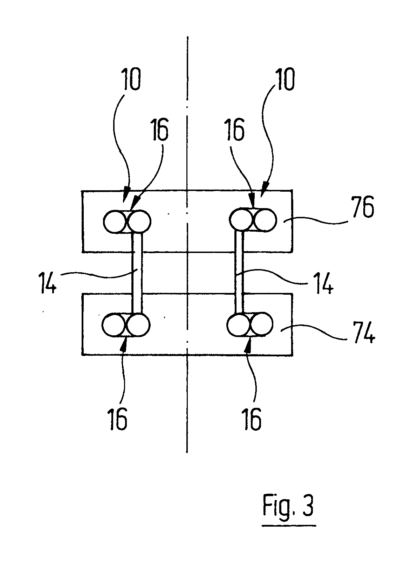

Ein Vergurtungssystem trägt insgesamt das Bezugszeichen 10 (vgl. Figur 3). Es umfaßt zwei als Pedikelschrauben ausgebildete Verankerungselemente 12, von denen in den Figuren 1 und 2 nur jeweils eine dargestellt ist, eine Verbindungsstange 14 und für jede Pedikelschraube 12 eine Verbindungseinrichtung 16. In Figur 1 ist ferner noch eine Führungsstange 18 und eine Führungshülse 20 dargestellt.

Die Pedikelschraube 12 hat einen zylindrischen Kopf 22, in dessen Stirnfläche 24 eine mittige axiale Stufenbohrung vorhanden ist, welche einen kleineren Durchmesser aufweisenden Gewindeabschnitt 26 und einen größeren Durchmesser aufweisenden Führungsabschnitt 28 aufweist. Gewindeabschnitt 26 und Führungsabschnitt 28 weisen an ihrem oberen Rand jeweils eine Einführschräge 30 auf. Die äußere Mantelfläche des Kopfes 22 der Pedikelschraube 12 ist als Außensechskant ausgeführt. Die Stirnfläche 24 ist mit einer strahlenförmig von der Mittelachse der Pedikelschraube 12 ausgehenden Riffelung (nicht sichtbar) versehen. In einem nicht dargestellten weiteren Ausführungsbeispiel ist der Außenmantel des Kopfes 22 der Pedikelschraube 12 ebenfalls mit einer Riffelung versehen.

Die Verbindungseinrichtung 16 umfaßt ein in der Draufsicht längliches Befestigungsteil 32 mit halbkreisförmig gerundeten Enden, welches einen Befestigungsabschnitt 34 und einen mit diesem einstückigen Lagerabschnitt 36 aufweist. In einem nicht dargestellten Ausführungsbeispiel ist der Lagerabschnitt gegenüber dem Befestigungsabschnitt um eine Achse verschwenkbar, welche in Einbaulage im wesentlichen senkrecht zur Längsachse der Pedikelschraube verläuft. In einer gewünschten Winkelposition ist ein derartiger Lagerabschnitt gegenüber dem Befestigungsabschnitt starr festlegbar. Der Befestigungsabschnitt 34 wird von einer Durchgangsbohrung 38 durchdrungen, welche in Einbaulage koaxial zur Längsachse der Pedikelschraube 12 liegt. Koaxial zur Durchgangsbohrung 38 weist der Befestigungsabschnitt 34 einen in den Figuren 1 und 2 oberen Gewindeansatz 40 und einen in den Figur 1 und 2 unteren Führungsvorsprung 42 auf. Der Außendurchmesser und die Höhe des Führungsvorsprungs 42 des Befestigungsabschnitts 34 des Befestigungsteils 32 entsprechen dem Außendurchmesser und der Höhe des Führungsabschnitts 28 im Kopf 22 der Pedikelschraube 12.

Der Durchmesser der Durchgangsbohrung 38 entspricht dem Durchmesser des Gewindeabschnitts 26 der Stufenbohrung im Kopf 22 der Pedikelschraube 12. Die in Einbaulage der Stirnfläche 24 des Kopfes 22 der Pedikelschraube 12 gegenüberliegende Fläche des Befestigungsabschnitts 34 des Befestigungsteils 32 weist ein zu der dort vorhandenen Riffelung komplementäre, in der Zeichnung jedoch nicht sichtbare Riffelung auf.

Der Lagerabschnitt 36 ist seitlich vom Befestigungsabschnitt 34 angeordnet. Er weist eine Gewindebohrung 44 auf, deren Längsachse parallel zur Durchgangsbohrung 38 verläuft. In den Figuren 1 und 2 von oben ist in den Lagerabschnitt 36 mittig durch die Gewindebohrung 44 eine im Querschnitt U-förmige Ausnehmung 46 eingebracht, die der Aufnahme der Verbindungsstange 14 dient und deren Weite daher dem Durchmesser der Verbindungsstange 14 entspricht. Die Mittelebene der Ausnehmung 46 steht senkrecht auf der durch die Längsachse der Durchgangsbohrung 38 im Befestigungsabschnitt 34 und die Längsachse der Gewindebohrung 44 im Lagerabschnitt 36 aufgespannten Ebene. Die U-förmige Ausnehmung 46 wird durch zwei Seitenwände 48 und einen zylindrisch gekrümmten Bodenabschnitt 50 begrenzt. Die U-förmige Ausnehmung 46 ist tiefer als die Gewindebohrung 44, derart, daß die Gewindebohrung 44 sich vom oberen Rand der U-förmigen Ausnehmung 46 bis nicht ganz auf jene Höhe erstreckt, in der die Seitenwände 48 in den Bodenabschnitt 50 übergehen.

An seinem in den Figuren 1 und 2 oberen Rand trägt der Lagerabschnitt 36 einen zur Gewindebohrung 44 koaxialen Gewindeansatz 52, welcher das gleiche Gewinde wie der Gewindeansatz 40 des Befestigungsabschnitts 34 trägt. In den Figuren 1 und 2 nach unten gesehen steht der Lagerabschnitt 36 über den Führungsvorsprung 42 des Befestigungsabschnitts 34 so über, daß er in Einbaulage in etwa auf Höhe des unteren Randes des Kopfs 22 der Pedikelschraube 12 endet. In seinem in den Figuren 1 und 2 unteren Bereich weist der Lagerabschnitt 36 eine in Einbaulage mit einer Fläche des Außensechskants auf der äußeren Mantelfläche des Kopfes 22 der Pedikelschraube zusammenarbeitende seitliche Anlagefläche 54 auf.

Die in Figur 1 dargestellte Führungsstange 18 trägt an ihrem in Figur 1 unteren Ende ein zum Gewindeabschnitt 26 der Stufenbohrung im Kopf 22 der Pedikelschraube 12 komplementäres Gewinde und ist in Figur 1 in diesen Gewindeabschnitt 26 eingeschraubt. Die in Figur 1 außerdem gezeigte Führungshülse 20 hat einen Innendurchmesser, welcher dem Außendurchmesser des Gewindeansatzes 40 des Befestigungsabschnitts 34 und des Gewindeansatzes 52 des Lagerabschnitts 36 entspricht. In der Innenwand der Führungshülse 20 ist an deren unterem Ende ein Gewinde 56 vorhanden, welches komplementär zum Gewinde an den Gewindeansätzen 40 bzw. 52 ist.

In Figur 2 sind schließlich noch eine Verbindungsschraube 58 und eine Klemmschraube 60 erkennbar. Die Verbindungsschraube 58 hat einen Kopf 62 mit kreisförmiger Außenkontur und einem Außendurchmesser, welcher unter Gleitspiel dem Innendurchmesser der Führungshülse 20 entspricht. Im Kopf 62 der Verbindungsschraube 58 ist eine Innensechskantausnehmung 64 vorhanden. Der Schraubabschnitt 66 der Verbindungsschraube 58 trägt ein zum Gewindeabschnitt 26 der Stufenbohrung im Kopf 22 der Pedikelschraube 12 komplementäres Gewinde und hat eine Länge, welche geringfügig kleiner ist als die Längserstreckung des Befestigungsabschnitts 34 vom oberen Rand des Gewindeansatzes 40 bis zum unteren Rand des Führungsvorsprungs 42 plus der Tiefe des Gewindeabschnitts 26 im Kopf 22 der Pedikelschraube 12.

Die Klemmschraube 60 verfügt über einen Kopf 68 mit einer Innensechskantausnehmung 70, der mit demjenigen der Verbindungsschraube 58 übereinstimmt. Der Schraubabschnitt 72 der Klemmschraube 60 trägt ein zu der Gewindebohrung 44 im Lagerabschnitt 36 komplementäres Gewinde.

Das Vergurtungssystem 10 kann folgendermaßen eingesetzt werden:

Zunächst wird in einem mikrochirurgischen Eingriff eine Hautinzision von ca. 4 cm vorgenommen und der Processus Spinosus zweier betroffener Wirbelkörper 74 und 76 (vgl. Figur 3) dargestellt. Die paravertebrale Muskulatur wird durchstoßen und der Bogen, der mediale Anteil der Facette und das Ligamentum flavum werden dargestellt. In einem hier nicht näher erläuterten Verfahren werden anschließend die genauen Positionen der einzubringenden Pedikelschrauben 12 festgelegt und markiert sowie ein Zugang zu den Pedikeln der beiden zu verbindenden Wirbelkörper 74 und 76 geschaffen. Ferner wird eine Bohrung durch den Pedikel durchgeführt und ein Gewinde in diese Bohrung eingeschnitten.

Dann wird ein in der Figur nicht dargestelltes Verlängerungselement mit einem Gewindezapfen in den Gewindeabschnitt 26 im Kopf 22 einer Pedikelschraube 12 eingeschraubt, bis er mit der Pedikelschraube 12 fest verbunden ist. Nun kann die Pedikelschraube 12 bequem an diesem Verlängerungselement gehalten und durch die kleine Hautöffnung und die paravertebrale Muskulatur hindurch in den Pedikel eingedreht werden. Die Länge dieses Verlängerungselementes ist so bemessen, daß dieses nach Eindrehen der Pedikelschraube 12 noch etwas über die Haut des Patienten nach außen vorsteht. Dieser Vorgang wird an allen notwendigen Pedikelstellen wiederholt, für die beiden benachbarten Wirbelkörper 74 und 76 an vier Stellen (diese werden nachfolgend unter Bezugnahme auf Figur 3 als links unten, links oben, rechts unten und rechts oben bezeichnet).

An den Verlängerungselementen wird nun ein in der Figur ebenfalls nicht dargestelltes Repositionsinstrument befestigt, mit dem sowohl die Relativstellung der betroffenen Wirbelkörper in ventrodorsaler Richtung als auch der Lordosewinkel eingestellt werden kann. Nach erfolgter Reposition werden das Repositionsinstrument und die Verlängerungselemente von den Pedikelschrauben 12 entfernt.

Nun verbindet der Operateur die Pedikel von Pedikelpaaren der benachbarten Wirbelkörper 74 und 76 mit Hilfe der Verbindungsstange 14 und der Verbindungseinrichtungen 16.

Hierzu werden zunächst mit Hilfe eines üblichen und bekannten Navigationssystems (nicht dargestellt) die exakten Positionen und Ausrichtungen der beiden Pedikelschrauben 12 an den Positionen links unten und links oben bestimmt. Die von diesem Navigationssystem ermittelten Daten werden nun an ein externes in der Zeichnung nicht dargestelltes Modell übertragen.

Bei diesem handelt es sich um eine Vorrichtung, in der die Relativpositionen zweier Pedikelschrauben mit zugehörigen Verbindungseinrichtungen zueinander simuliert werden können, indem eine (ggf. simulierte) Pedikelschraube in entsprechenden Führungen durch Mikrometerschrauben entlang der drei Raumachsen translatorisch und winkelmäßig verstellt und so in eine dem tatsächlichen Operationsgebiet entsprechende Relativposition gegenüber der anderen Pedikelschraube gebracht werden kann. Eine Verbindungsstange wird nun mittels einer entsprechenden Vorrichtung so gebogen, daß sie in die Verbindungseinrichtungen der im Modell befindlichen Pedikelschrauben und damit auch in diejenigen der im Patienten gesetzten Pedikelschrauben paßt.

Auf diese Weise wird also zunächst eine Verbindungsstange 14 entsprechend den tatsächlich auf der linken Seite des Operationsgebietes vorhandenen Gegebenheiten zurechtgebogen.

In den Gewindeabschnitt 26 der Stufenbohrung im Kopf 22 der linken oberen Pedikelschraube 12 wird nun eine Führungsstange 18 eingeschraubt. Auf diese wird dann ein Befestigungsteil 32 einer Verbindungseinrichtung 16 mit Hilfe der im Befestigungsabschnitt 34 vorhandenen Durchgangsbohrung 38 aufgefädelt und bis zum Kopf 22 der Pedikelschraube 12 abgesenkt. Dabei greift der Führungsvorsprung 42 des Befestigungsabschnitts 34 des Befestigungsteils 32 in den Führungsabschnitt 28 der Stufenbohrung im Kopf 22 der Pedikelschraube 12 ein, was durch die am oberen Rand der Stufenbohrung im Kopf 22 der Pedikelschraube 12 vorhandene Einführschräge 30 erleichtert wird.

Die seitliche Anlagefläche 54 am Lagerabschnitt 36 kommt dabei in Anlage an eine Fläche des Außensechskants am Kopf 22 der Pedikelschraube 12, wodurch deren Relativ-Winkelposition festliegt. Eine zusätzliche Sicherung bieten die am Kopf 22 der Pedikelschraube 12 und am Befestigungsabschnitt 34 des Befestigungsteils 32 vorhandenen komplementären Riffelungen. Nun wird über die Führungsstange 18 eine Führungshülse 20 aufgeschoben und mit ihrem Gewinde 56 mit dem Gewindeansatz 40 des Befestigungsabschnitts 34 des Befestigungsteils 32 verschraubt. Eine solche Situation ist in Figur 1 dargestellt.

Die Führungsstange 18 wird nun gelöst und herausgezogen, wobei mit der Führungshülse 20 gegengehalten wird. Eine Verbindungsschraube 58 wird in der Führungshülse 20 in Richtung auf das Befestigungsteil 32 abgesenkt. Dadurch, daß der Durchmesser des Kopfs 62 der Verbindungsschraube 58 im Gleitspiel mit dem Innendurchmesser der Führungshülse 20 zusammenarbeitet, wird die Verbindungsschraube 58 während des Absenkens exakt geführt, so daß sichergestellt ist, daß sie durch die Durchgangsbohrung 38 im Befestigungsabschnitt 34 des Befestigungsteils 32 hindurch in den Gewindeabschnitt 26 im Kopf 22 der Pedikelschraube 12 eingreifen kann. Die Verbindungsschraube 58 wird mit einem Sechskantschlüssel, welcher in die Sechskantausnehmung 64 in ihrem Kopf 62 eingreift, lose verschraubt.

Ein Ende der vorgebogenen Verbindungsstange 14 wird nun außerhalb des Körpers des Patienten in die U-förmigen Querschnitt aufweisende Ausnehmung 46 eines zweiten Befestigungsteils 32 so eingeführt, wie es der im Modell simulierten Situation entspricht. In der U-förmigen Ausnehmung 46 wird die Verbindungsstange 16 durch Festschrauben einer Klemmschraube 60 festgelegt. Die so geschaffene Einheit aus Verbindungseinrichtung 16 (Befestigungsteil 32 und Klemmschraube 60) und Verbindungsstange 14 wird jetzt auf eine zuvor in die Pedikelschraube 12 links unten eingeschraubte Führungsstange 18 mit Hilfe der Durchgangsbohrung 38 im Befestigungsabschnitt 34 des Befestigungsteils 32 aufgefädelt.

Wie bereits bei der Position links oben wird auch hier durch die Führungsstange 18 und die mit dieser im Gleitspiel zusammenarbeitende Durchgangsbohrung 38 im Befestigungsabschnitt 34 des Befestigungsteils 32 ein sicheres und einfaches Einführen der Verbindungseinrichtung 16 gewährleistet.

Das Einführen des Führungsvorsprungs 42 des Befestigungsabschnitts 34 des Befestigungsteils 32 in den Führungsabschnitt 28 der Stufenbohrung im Kopf 22 der Pedikelschraube 12 wird auch hier durch die Einführschräge 30 am oberen Rand des Führungsabschnitts 28 des Kopfes 22 der Pedikelschraube 12 erleichtert. Da die Verbindungsstange 14 ja bereits in der notwendigen Weise abgelängt und zurechtgebogen ist, kommt ihr anderes Ende in der U-förmigen Ausnehmung 46 des linken oberen Befestigungsteils 32 zum Liegen.

Nun wird beim linken unteren Befestigungsteil 32 eine Führungshülse 20 auf den Gewindeansatz 40 des Befestigungsabschnitts 34 des Befestigungsteils 32 aufgeschraubt, die Führungsstange 18 entfernt und, wie oben im Zusammenhang mit dem linken oberen Befestigungsteil 32 beschrieben, die Verbindungsschraube 58 eingeführt und lose verschraubt.

Am linken oberen Befestigungsteil 32 wird die Führungshülse 20 vom Gewindeansatz 40 des Befestigungsabschnitts 34 des Befestigungsteils 32 entfernt und auf den Gewindeansatz 52 des Lagerabschnitts 36 aufgeschraubt. Nun wird eine Klemmschraube 60 in die Führungshülse 20 eingeführt, wobei aufgrund des mit der Führungshülse 20 im Gleitspiel zusammenarbeitenden Kopfes 68 der Klemmschraube 60 eine relativ exakte Führung während des Absenkens der Klemmschraube 60 in die Gewindebohrung 44 im Lagerabschnitt 36 gewährleistet ist (s.o.). Darüber hinaus erleichtern die Einführschrägen 30 an den oberen Rändern der Gewindebohrung 44 im Lagerabschnitt 36 das Einführen der Klemmschraube 60.

Über einen Sechskantschlüssel, der in die Sechskantausnehmung 70 im Kopf 68 der Klemmschraube 60 eingreift, wird die Klemmschraube 60 soweit gedreht, bis sie am entsprechenden Ende der Verbindungsstange 14 lose anliegt. Diese Situation ist in Figur 2 dargestellt.

Beide Befestigungsteile 32, also jenes an der linken unteren als auch jenes an der linken oberen Position, sind zum gegenwärtigen Zeitpunkt noch mit einer Führungshülse 20 verbunden, die durch die Hautinzisionen nach außen über die Haut des Patienten übersteht. Mit diesen Führungshülsen 20 kann nun der Operateur, falls erforderlich, noch eine Feinjustierung der Relativposition der beiden zu verbindenden Wirbelkörper 74 und 76 vornehmen. Sobald die endgültige Position erreicht ist, wird einerseits die Verbindungsschraube 58 am linken unteren Befestigungsteil 32 und andererseits die Klemmschraube 60 am linken oberen Befestigungsteil 32 festgezogen. Anschließend werden die Innensechskantschlüssel und die entsprechenden Führungshülsen 20 entfernt. Die beiden Wirbelkörper 74 und 76 sind nun auf ihrer linken Seite starr miteinander verbunden.

Der gleiche Vorgang wiederholt sich nun auf der rechten Seite, wo zunächst jedoch von den dortigen Pedikelschrauben 12 noch das Repositionsinstrument abgenommen werden muß.

Schließlich sind die beiden Wirbelkörper 74 und 76 insgesamt über vier Pedikelschrauben 12, vier Verbindungseinrichtungen 18 und zwei Verbindungsstangen 14 miteinander starr verbunden. Dies ist schematisch in Figur 3 dargestellt.

## Patentansprüche

1. Vergurtungssystem zur Spondylodese der Lendenwirbelsäule mit
a) mindestens zwei jeweils in einem Pedikel verankerbaren Verankerungselementen;
b) mindestens einer zwischen zwei benachbarten Wirbelkörpern verlaufenden Verbindungsstange; und
c) für jedes Verankerungselement einer über ein Befestigungselement an diesem befestigbaren Verbindungseinrichtung für die Verbindungsstange;
**dadurch gekennzeichnet, daß**
d) jede Verbindungseinrichtung (16) ein mit dem Verankerungselement (12) in axialer Richtung verbindbares Befestigungsteil (32) aufweist, das einen seitlichen Lagerabschnitt (36) besitzt, in dem ein Endabschnitt der Verbindungsstange (14) fest aufgenommen werden kann.

2. Vergurtungssystem nach Anspruch 1, **dadurch gekennzeichnet, daß** der seitliche Lagerabschnitt (36) des Befestigungsteils (32) eine in Einbaulage in axialer Richtung des Verankerungselements (12) gesehen offene im Querschnitt U-förmige Ausnehmung (46) zur Aufnahme des Endabschnitts der Verbindungsstange (14) aufweist und ein in Einbaulage in axialer Richtung des Verankerungselements (12) einsetzbares Klemmelement (60) vorgesehen ist, mit dem die Verbindungsstange (14) in der Uförmigen Querschnitt aufweisenden Ausnehmung (46) verklemmt werden kann.

3. Vergurtungssystem nach Anspruch 2, **dadurch gekennzeichnet, daß** das Klemmelement als Klemmschraube (60) ausgebildet ist, die mit einem in die Seitenwände (48) der U-förmigen Querschnitt aufweisenden Ausnehmung (46) eingebrachten und sich in Einbaulage parallel zur Längsachse des Verankerungselements erstreckenden Gewinde (44) zusammenarbeitet.

4. Vergurtungssystem nach einem der vorhergehenden Ansprüche, **dadurch gekennzeichnet, daß** daß eine Führungsstange (18) mit dem Verankerungselement (12) verbindbar ist.

5. Vergurtungssystem nach Anspruch 4, **dadurch gekennzeichnet, daß** das Befestigungsteil (32) eine Durchgangsbohrung (38) aufweist, die mit der Führungsstange (18) im Gleitspiel zusammenarbeiten kann.

6. Vergurtungssystem nach einem der vorhergehenden Ansprüche, **dadurch gekennzeichnet, daß** das Befestigungsteil (32) mindestens einen Ansatz (40, 52) aufweist, an dem eine Führungshülse (20) befestigbar ist.

7. Vergurtungssystem nach Anspruch 6, **dadurch gekennzeichnet, daß** das Befestigungselement (58) und/oder das auf die Verbindungsstange (14) arbeitende Klemmelement (60) mindestens abschnittsweise im Gleitspiel so mit der Führungshülse (20) zusammenarbeiten, daß sie durch diese zum Verankerungselement (12) bzw. zur U-förmigen Querschnitt aufweisenden Ausnehmung (46) geführt werden können.

8. Vergurtungssystem nach einem der vorhergehenden Ansprüche, **dadurch gekennzeichnet, daß** an mindestens einer in Einbaulage in axialer Richtung des Verankerungselements (12) nach außen zeigenden Kante des Verankerungselements (12) und/oder des Befestigungsteils (32) eine Einführschräge (30) vorgesehen ist.

9. Vergurtungssystem nach einem der vorhergehenden Ansprüche, **dadurch gekennzeichnet, daß** das Befestigungsteil einen Befestigungsabschnitt aufweist und der Lagerabschnitt gegenüber dem Befestigungsabschnitt um eine Achse verschwenkbar ist, welche in Einbaulage im wesentlichen senkrecht zur Längsachse des Verankerungselements liegt, und in einer gewünschten Winkelposition gegenüber dem Befestigungsabschnitt festlegbar ist.
